# EUROPÄISCHE PATENTANMELDUNG

(11) **EP 1 473 358 A2**
(43) Veröffentlichungstag der Anmeldung: **03.11.2004**
(21) Anmeldenummer: 04090164.7
(22) Anmeldetag: 23.04.2004
(51) Int. Cl.: C12M 1/06, C12M 1/08, B01F 3/04

(54) **Verfahren und Vorrichtung zum Begasen und Rühren von Stoffen**

(30) Priorität: 30.04.2003 DE 10320690; 22.04.2004 DE
(71) Anmelder: Chemie- und Tankanlagenbau Reuther GmbH, 15517 Fürstenwalde (DE)
(72) Erfinder: Reuther, Carl-Friedrich, Dr., 14593 Berlin (DE)
(74) Vertreter: Hannig, Wolf-Dieter, Dipl.-Ing.

(57) **Zusammenfassung**

Die Erfindung betrifft ein Verfahren zum Begasen und Ruhren von Nährlösungen für Produktbildner wie Mikroorganismen, Zellen u. dgl., bei dem die Nährlösung in einem geschlossenen Reaktorraum eingebracht, mit einer unter Druck stehenden Gasmenge, beispielsweise Luft, Sauerstoff o. dgl., beaufschlagt und die Nährlösung zugleich gerührt wird, wobei das Gas in vom Flussigkeitsspiegel verschieden hohen vertikalen Flüssigkeitssäulen (Begasungsebenen) in die Nährlösung des Reaktorraums über Begasungskorper zugeführt wird sowie Gas und Nährmittel durch im Reaktorraum rotierendes Rührkörper durchmischt werden.

Der Erfindung liegt die Aufgabe zugrunde, die Begasung signifikant zu vergleichmäßigen, den Wirkungsgrad unter gleichzeitiger Verringerung der Rührgeschwindigkeit und Einsparung von Energie zu erhohen.

Gelöst wird diese Aufgabe dadurch, dass die Menge und der Druck **(p**_{**G**}**)** des zuzufuhrenden Gases in Abhängigkeit des in den einzelnen Begasungsebenen **(I;II;III)** wirkenden hydrostatischen Druckes **(p**_{**t**}**)** und der Stoffeigenschaften der Nährlösung dynamisch oder statisch gesteuert wird.

## Beschreibung

Die Erfindung betrifft ein Verfahren zum Begasen und Rühren von Stoffen wie Flüssigkeiten, fließfähige Feststoffe, Nährlösungen für Produktbildner, beispielsweise Mikroorganismen, Zellen u. dgl., bei dem der Stoff in einem Reaktorraum eingebracht, mit einer unter Druck stehenden Gasmenge, beispielsweise Luft, Sauerstoff o. dgl., beaufschlagt und zugleich gerührt wird, wobei das Gas in vom Stoffspiegel verschieden hohen vertikalen Stoffsäulen (Begasungsebenen) in den Stoff des Reaktorraums über Begasungskörper zugeführt wird sowie Gas und Stoff durch im Reaktorraum rotierendes Rührkörper durchmischt werden.

Die Erfindung betrifft ferner eine Vorrichtung zum Begasen und Rühren von Stoffen wie Flüssigkeiten, fließfähige Feststoffe, Nährlösungen für Produktbildner, beispielsweise Mikroorganismen, Zellen u. dgl., mit einem Behältnis als Reaktor zur Aufnahme des Stoffes, einer in das Behältnis geführten Rührwelle mit an dieser befestigten Rührkörpern und

Begasungskörpern, wobei die Begasungskörper in vom Stoffspiegel des Stoffes verschieden beabstandeten Begasungsebenen angeordnet sind, und einer Gaserzeugungseinheit, die Druckgas in die Begasungskörper zur Begasung des Stoffes fördert.

Die Erfindung betrifft auch eine Vorrichtung zum Begasen und Rühren von Stoffen wie Flüssigkeiten, fließfähige Feststoffe, Nährlösungen für Produktbildner beispielsweise Mikroorganismen, Zellen u. dgl., mit einem Behältnis als Reaktor zur Aufnahme des Stoffes, einer in den Behältnis geführten hohlen Antriebswelle mit an dieser befestigten Rührelementen und Begasungselementen, wobei die Begasungselemente in vom Flüssigkeitsspiegel der Nährlösung verschieden beabstandeten Begasungsebenen angeordnet sind, und einer Gaserzeugungseinheit, die Druckgas in die Begasungskörper zur Begasung des Stoffes fördert.

Aus der CH-A 677 496 bzw. EP-B1 0 365 621 ist eine Vorrichtung zum submersen Kultivieren von pflanzlichen, tierischen und humanen Gewebezellen bekannt, die aus einem sterilisierbaren Behälter mit einer zentral angeordneten Hohlwelle mit mindestens einer Schikane zwischen zwei Rührelementen und/oder mit einem oder mehreren übereinander angeordneten Rührelementen und Schikanen. Die Schikanen sind jeweils zwischen zwei Rührelementen angeordnet, die mit einem gasundurchlässigen Teil und einem gasdurchlässigen Teil versehen sind. Der gasdurchlässige Teil besteht aus einem gelochten Blech, aus einem porösen Material, vorzugsweise Sintermaterial, Keramik, Kunststoff oder semipermeable Membran. Die Schikanen sind an einem herausnehmbaren Schikanenkorb befestigt, wobei die Schikanen und der Schikanenkorb als Hohlkörper ausgebildet sind. Ein Teil der Schikanen ist gasdurchlässig ausgebildet.
Der auf den Schikanen lastende hydrostatische Druck der Flüssigkeitssäule nimmt bei dieser bekannten Lösung mit zunehmender Tauchtiefe drastisch zu, so dass das zugeführte Gas bei seinem Austritt aus den Schikanen einen immer größeren Druck überwinden muss. Dies führt zu einer vollkommen ungleichmäßigen Begasung der Nährlösung, wodurch das Wachstum der Gewebezellen entsprechend nachteilig beeinflusst wird. Der erreichte Wirkungsgrad ist gering und der Energieverbrauch verhältnismäßig hoch.

Bei diesem Stand der Technik liegt der Erfindung die Aufgabe zugrunde, die Begasung signifikant zu vergleichmäßigen, den Wirkungsgrad unter gleichzeitiger Verringerung der Rührgeschwindigkeit und Einsparung von Energie zu erhöhen.

Diese Aufgabe wird durch die kennzeichnenden Merkmale des Anspruchs 1 und der nebengeordneten Ansprüche 12 und 16 gelöst.
Vorteilhafte Ausgestaltungen des erfindungsgemäßen Verfahrens und der Vorrichtungen sind den Unteransprüchen entnehmbar.

Das erfindungsgemäße Verfahren überwindet die unterschiedlichen hydrostatischen Drücke in den einzelnen Begasungsebenen durch eine Steuerung der Gaszuführung in einer entsprechenden Menge und mit einem geringfügig über den hydrostatischen Druck liegenden Gasdruck. Hierdurch ist über die gesamte Reaktorhöhe eine weitgehend gleichmäßige Verteilung des Gases in Form von kleinen sauerstoffreichen Gasblasen erreichbar, die eine gute Sauerstoffversorgung der Produktbildner gewährleisten.
Trotz Herabsetzung der Umdrehungsgeschwindigkeit des Rührers werden Absetzvorgänge sicher verhindert und die Durchmischung verbessert.
Ein weiterer Vorteil der erfindungsgemäßen Lösung besteht darin, dass infolge der geringeren Umdrehungsgeschwindigkeit die Scherkräfte an den Rührelementen herabgesetzt werden, wodurch sich eine überaus schonende Behandlung der Produktbildner ergibt.
Die Erfindung ist insbesondere für den Stoff- und Gasaustausch in der Pharmazie, Biotechnologie, Chemie sowie Nahrungs- und Genussmittelindustrie und Wasserbehandlung geeignet.

Die Erfindung soll nachstehend an mehreren Ausführungsbeispielen näher erläutert werden.
Es zeigen:
- Fig. 1: eine Prinzipdarstellung des erfindungsgemäßen Verfahrens einschließlich Vorrichtung mit außerhalb des Behältnis gelegener Ansteuerung der Gaszufuhr in die einzelnen Begasungsebenen,
- Fig. 2: eine weitere Variante gemäß Fig. 1 mit separater Gaszuführung durch in die Rührwelle eingebrachter Gaskanäle und
- Fig. 3: eine weitere Ausführungsform des erfindungsgemäßen Verfahrens und der Vorrichtung mit innerhalb des Behältnis erfolgender Ansteuerung der Gaszufuhr in die Begasungsebenen.

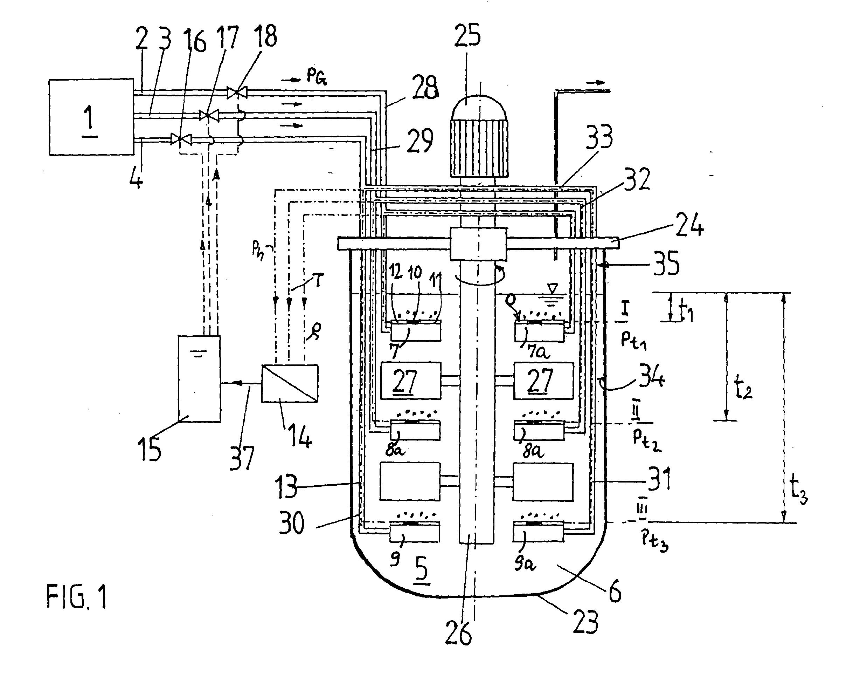

Fig. 1 stellt das Grundprinzip des erfindungsgemäßen Verfahrens in einer Ausführungsvariante dar. Von einer Gaserzeugungseinheit **1,** die das unter Begasungsdruck **p**_{**G**} stehende Gas, beispielsweise Luft, liefert, führen Gasleitungen **2, 3** und **4** zu in unterschiedlicher Tiefe **t**_{**1**}**, t**_{**2**}**, t**_{**3**} des Reaktorraumes **5** abgesenkten horizontalen Begasungskörpern **7, 8** und **9** in den zu behandelnden Stoff, beispielsweise eine Nährlösung 6. Die Masse des Gases wird mittels separater Durchflussmesser 50 ermittelt, die an die jeweiligen Gasleitungen **2, 3** und **4** angeschlossen sind. Die Messung des Gasdruckes **p**_{**G**} erfolgt mit entsprechend angeschlossenen Druckmessern **51.**
Die Begasungskörper **7, 8** und **9** sind zumindest an ihrer Oberseite **O** gasdurchlässig und tragen an bzw. auf der Oberseite **O** Sensoren **10** für die Ermittlung des hydrostatischen Druckes der über dem jeweiligen Begasungskörper lastenden Stoffsäule, einen Sensor **11** für die Temperatur und einen Sensor **12** für die Stoffwerte der Nährlösung **6**. Die Sensoren **10, 11** und **12** sind mit Verbindungsleitungen **13,** die an den Gasleitungen **2, 3** oder **4** verlegt sind, mit einem Analog-Digital-Wandler **14** verbunden, der die Sensorsignale in digitale Signale umsetzt und einem Prozessor **15** zuführt.
Die Messwerte des Gasdurchflusses und des Gasdruckes werden ebenso dem Prozessor zugeleitet.
Der Prozessor **15** verarbeitet die Signale und steuert in Abhängigkeit des in jeder Begasungsebene **I, I** und **III** ermittelten tatsächlichen Druckes **p**_{**t1**}**, p**_{**t2**} und **p**_{**t3**}, der Stoffeigenschaften der Nährlösung, beispielsweise Viskosität, Dichte und Temperatur der Nährlosung die mit den Gasleitungen **2, 3** und **4** strömungsseitig verbundenen Stellventile **16, 17** und **18** so an, dass jede Begasungsebene **I, II** und **III** mit einer Gasmenge beaufschlagt wird, deren Druck **p**_{**G**} in der Lage ist, den tatsächlichen Druck **p**_{**t**} in den einzelnen Begasungsebenen zu überwinden.
Dadurch ist es möglich, die Gaszufuhr auf die sich ändernden Bedingungen in der Nährlösung, beispielsweise auf die sich ändernde Viskosität oder Dichte infolge des Wachstums der Zellen, Mikroorganismen o. dgl. dynamisch anzupassen.
Es gehört natürlich auch zum erfindungsgemäßen Verfahren, wenn auf den Einsatz der Sensoren **10, 11** und **12** verzichtet und das Verfahren statisch ausgeführt wird. Da die Tauchtiefen **t**_{**1**}**, t**_{**2**} und **t**_{**3**} der Begasungskörper **7, 8** und **9** bzw. **7a, 8a** und **9a** im Behältnis **23** konstruktiv fest liegen und sich während des Prozesses nicht verändern, kann von einem annähernd konstanten tatsächlichen Druck **p**_{**t**} in der jeweiligen Begasungsebene ausgegangen werden. Die Regelventile **16, 17** und **18** werden für diesen Fall in Abhängigkeit des ermittelten tatsächlichen Druckes fest voreingestellt, so dass die Begasungsebenen **I, II** und **III** mit einer vorgegebenen Gasmenge gleichen Druckes beaufschlagt werden, der annähernd über dem festgestellten tatsächlichen Druck gehalten wird.
Die Vorrichtung für die Durchführung des erfindungsgemäßen Verfahrens besteht im Wesentlichen aus einem den Reaktorraum **5** bildenden, senkrecht stehenden zylindrischen Behältnis **23,** der durch einem Deckel **24** verschlossen ist. Auf dem Deckel **24** ist ein Antriebsaggregat **25** angeflanscht, dessen massive Rührwelle **26** abgedichtet durch den Deckel **24** geführt und zentrisch in das Behältnis **23** hineinreicht. Der Deckel **24** ist zum Abführen des Gases aus der Nährlösung mit einer nach außen führenden nicht dargestellten Abzugsleitung versehen.
Die Rührwelle **26** trägt Rührkörper **27**, die senkrecht von der Rührwelle **26** aufragen und übereinander im Abstand voneinander angeordnet sind.
Zwischen den Rührkörpern **27** befinden sich hohle, horizontal angeordnete Begasungskörper **7, 8, 9** und **7a, 8a, 9a**, die unterschiedlich tief in die eingefüllte Nährlösung **6** des Behältnis **23** eintauchen und die Begasungsebenen **I, II** und **III** mit den Tauchtiefen **t**_{**1**}**, t**_{**2**} und **t**_{**3**} definieren.
Jeder der Begasungskörper **7, 8** und **9** bzw. **7a, 8a** und **9a** besitzt eine separate Zuleitung **28, 29, 30, 31, 32** und **33** die entlang des inneren Mantels **34** verlegt einen Begasungskorb **35** bilden und durch den Deckel **24** geführt sind. Oberhalb des Deckels **24** werden die Zuleitungen **31, 32** und **33** mit den Zuleitungen **28, 29** und **30** zusammengeführt, die ihrerseits über Regelventile **16, 17** und **18** mit der Gaserzeugungseinheit **1** verbunden sind, welche das Gas in die Zuleitungen **28, 29**, **30, 31, 32** und **33** zur Begasung der einzelnen Begasungsebenen **I, II** und **III** fördert.
Die Begasungskörper **7, 8, 9** und **7a, 8a, 9a** haben an ihrer Oberseite **O** Öffnungen **36** zum Durchtritt des zugeführten Gases in die Nährlösung **6.** Je nach Art und den Stoffeigenschaften der Nährlösung können diese Öffnungen **36** unterschiedliche Durchmesser und Gestalt besitzen, um die Form der Gasblasen entsprechend zu verändern. Zweckmäßigerweise weisen deshalb die Begasungskörper **7, 8, 9** und **7a, 8a, 9a** einen in die Oberseite **O** montierten, aber auswechselbaren Einsatz auf, in dem die Öffnungen, beispielsweise Bohrungen, eingebracht sind.
Jedem Rührkörper **27** sind jeweils zwei Begasungskörpern **7** und **8, 8** und **9, 7a** und **8a** sowie **8a** und **9a** zugeordnet. Sie gewährleisten eine gute Durchmischung der Nährlösung **6**, wodurch eine Absetzung von schwereren Bestandteilen der Nährlösung verhindert und eine moglichst lange Stoffübertragungszeit von den Gasblasen in die Nährlösung mit der erforderlichen Strömungsgeschwindigkeit und Turbulenzen in der Nährlösung sowie der Transport des gelösten Gases in den Kern der Flüssigkeit erreicht wird.

Die Sensoren **10, 11** und **12** sind durch Verbindungsleitungen **13,** die entlang der Zuleitungen **28, 29, 30, 31, 32** und **33** verlegt und durch den Deckel **24** geführt sind, mit einem Analog/Digital Wandler **14** verbunden, der die Sensorsignale über eine Verbindungsleitung **37** an einen Prozessor **15** zur Verarbeitung weiterleitet.
Es gehört selbstverständlich zur erfindungsgemäßen Lösung, wenn die separaten Zuleitungen **28, 29, 30, 31, 32** und **33** am Außenmantel des Behältnis **23** verlegt und durch den Mantel des Behältnis **23** hindurchgeführt werden. Die Zuleitungen **28, 29**, **30, 31, 32** und **33** enden in den horizontal angeordneten, hohlen Begasungskörpern **7, 8, 9** bzw. **7a, 8a** und **9a** innerhalb des Reaktorraumes **5** in den beschriebenen Begasungsebenen **I**, **II** und **III**.

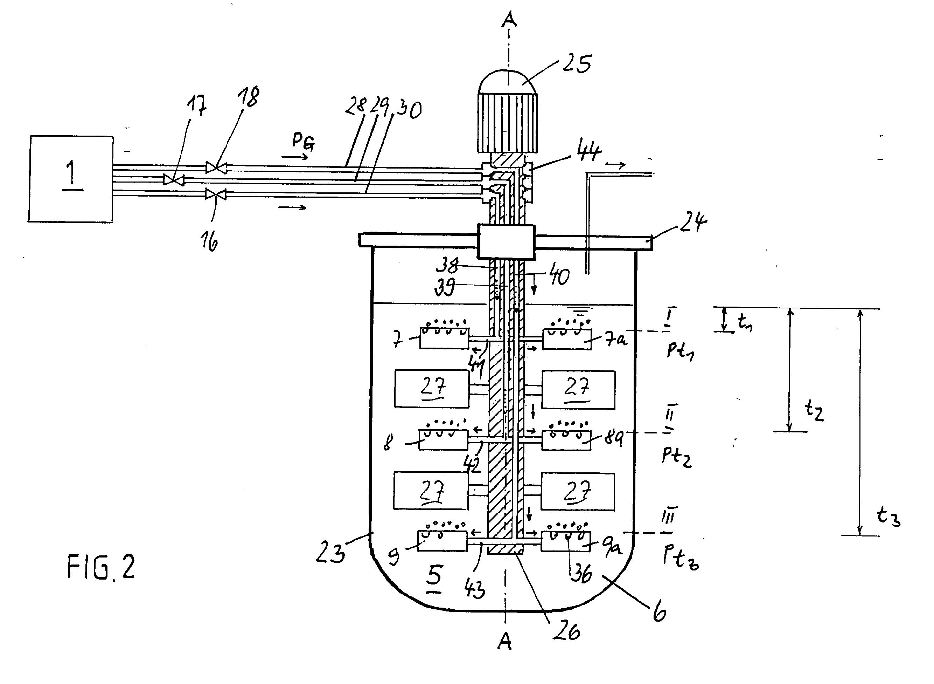

Fig. 2 zeigt eine weitere Variante der erfindungsgemäßen Vorrichtung mit separater Gaszuführung zu den einzelnen Begasungsebenen **I, II** und **III.**
Im Gegensatz zur Ausführung gemäß Fig. 1 rotieren die Begasungskörper **7, 8, 9** bzw. **7a, 8a, 9a** mit den Rührkorpern **27** mit der Rührwelle **26.** Dazu sind die Begasungskörper **7** und **7a, 8** und **8a** sowie **9** und **9a** mit der Rührwelle **26** in Höhe der Begasungsebenen **I, II** und **III** fest verbunden. Teilweise verlaufen die Zuleitungen **28, 29** und **30** in der Rührwelle **26** als parallel zueinander angeordnete Gaskanäle **38, 39** und **40** weiter, von denen in der jeweiligen Begasungsebene **I, II** und **III** senkrecht zur Rührwellenachse **A-A** angeordnete Abzweigkanäle **41, 42** und **43** abgehen, die zu den jeweiligen Begasungskörpern **7** und **7a, 8** und **8a, 9** und **9a** führen. Die Rührwelle **26** ist auch bei dieser Ausführung abgedichtet durch den Deckel **24** geführt. Auf der Rührwelle **26** sitzt eine Drehdurchführung **44,** durch die sichergestellt wird, dass die Gaskanäle **38, 39** und **40** druck- und gasdicht mit den Zuleitungen **28, 29** und **30** verbunden werden können, die über die beschriebenen Regelventile **16, 17** und **18** mit der Gaserzeugungseinheit **1** strömungsseitig in Verbindung stehen. Da die Einbaulagen der horizontalen Begasungskörper **7** und **7a , 8** und **8a** sowie **9** und **9a** im Behältnis **23** konstruktiv festliegen, werden die Regelventile **16, 17** und **18** auf den in den einzelnen Begasungsebenen **I, II** und **II** wirkenden hydrostatischen Druck abzugebenen Gasdruck voreingestellt.
Natürlich ist es auch moglich, die Sensoren **10, 11** und **12** in der Tauchtiefe **t**_{**1**}**, t**_{**2**} und **t**_{**3**} nahe der Begasungskörper am Innenmantel des Behältnis **23** fest zu haltern. Dies wird zweckmaßigerweise dadurch realisiert, dass die Sensoren 10, 11 und 12 von außen mittels einer nicht dargestellten Flanschverbindung durch den Mantel des Behältnis geführt sind. Von den Sensoren **10, 11** und **12** führen dann die Verbindungsleitungen **13** zum A/D-Wandler **14,** der die gelieferten Signale zum Prozessor 15 weiterleitet. Die Ansteuerung der Regelventile **16, 17** und **18** entspricht der der Ausführung gemaß Fig. 1.

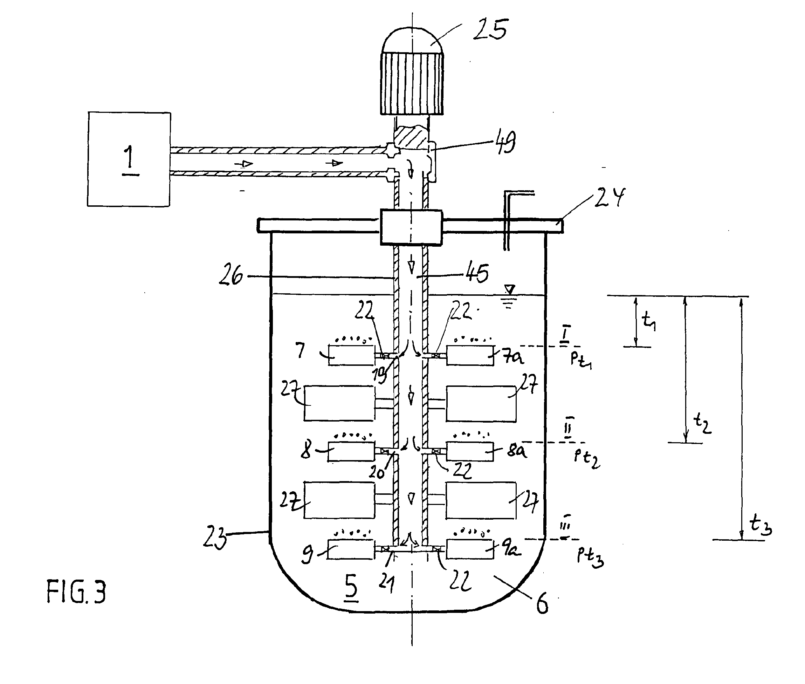

Fig. 3 zeigt das Grundprinzip des erfindungsgemäßen Verfahrens mit einer innerhalb des Reaktorraumes **5** liegenden Ansteuerung der Gaszufuhr in die Begasungsebene **I, II** und **III.** In den Abzweigleitungen **19, 20, 21** zu den Begasungskörpern **7, 7a, 8, 8a, 9, 9a** befinden sich Regelorgane **22,** beispielsweise Ventile, Druckminderer, Klappen oder Blenden, die sicherstellen, dass die hohlen Begasungselemente **7, 8** und **9** mit einer Gasmenge beaufschlagt wird, deren Druck **p**_{**G**} geringfügig größer ist als der jeweilige hydrostatische Druck der entsprechenden Begasungsebene.
Die Regelorgane **22** können jedoch auch, ohne die Erfindung zu verlassen, entsprechend des in den einzelnen Tauchtiefen **t**_{**1**}**, t**_{**2**} und **t**_{**3**} lastenden hydrostatischen Druckes durch einfache Öffnungen ersetzt werden, deren Durchmesser so gewählt ist, dass die hohlen Begasungskörper **7, 8** und **9** mit einer Gasmenge versorgt werden, deren Druck konstant geringfügig über den jeweiligen hydrostatischen Druck liegt.
Es ist aber auch möglich die hohlen Begasungskörper **7, 8** und **9** mit porösem offenporigen Material auszufüllen, dessen wirksame Oberfläche so gewählt wird, das ein Gasdruck zur Überwindung des hydrostatischen Druckes in den einzelnen Begasungsebenen erreicht wird.
Durch einen zentralem Gaszuführungskanal **45** wird das Druckgas der als Hohlwelle ausgebildete Rührwelle **26** zugeführt. Von der Hohlwelle zweigen die senkrecht abgehende Abzweigleitungen **19, 20** und **21** für das Gas ab, die die Begasungskörper **7** und **7a, 8** und **8a** sowie **9** und **9a** in den einzelnen Begasungsebenen **I, II** und **III** mit dem zentralen Gaszuführungskanal **45** verbinden. Die Hohlwelle **26** ist mittels einer Drehdurchführung **49** mit der Gaserzeugungseinheit **1** druck- und gasdicht verbunden.
Die Stellorgane **22** in den Abzweigleitungen **19, 20** und **21** werden jeweils auf den gewünschten Begasungsdruck **p**_{**G**} voreingestellt, so dass der Begasungsdruck in jeder Begasungsebene etwas größer ist als der jeweilige tatsächlich wirkende Druck.
Ohne die Erfindung zu verlassen, versteht es sich, dass die horizontalen Begasungskörper eine unterschiedliche Länge haben, eine hydrodynamisch angepasste Form aufweisen und sich mit zunehmenden Abstand von der Rührwelle **26** verjüngen können. Die Rührkörper **27** und Begasungskörper **7, 7a, 8, 8a, 9** und **9a** können auch zueinander winklig versetzt an der Rührwelle **26** angeordnet sein. Ebenso können die Rührkörper **27** einen Anstellwinkel zur Drehrichtung besitzen, um in verschiedenen Höhen der Nährflüssigkeit im Behältnis entsprechend angepasste Strömungsverhältnisse zu realisieren.

Die Erfindung ist auch nicht an die im Ausführungsbeispiel dargestellten drei Begasungsebenen **I, II** und **III** gebunden. Je nach den Erfordernissen wird die Anzahl der Begasungsebenen entsprechend variieren.
Es lassen sich beispielsweise auch die Begasungskörper ohne weiteres mit einem klassischen Scheibenrührer kombinieren.
Das Behältnis **23** kann in liegender oder stehender bzw. in schräger Bauweise angeordnet sein und die Rührwelle vom Boden oder vom Deckelbereich aus angetrieben werden.

### Aufstellung der verwendeten Bezugszeichen

| | |
|---|---|
| Gaserzeugungseinheit | 1 |
| Gasleitungen | 2, 3, 4 |
| Reaktorraum | 5 |
| Nährlösung | 6 |
| horizontale Begasungskörper | 7,7a,8,8a,9,9a |
| Sensor für hydrostatischen Druck | 10 |
| Sensor für Temperatur | 11 |
| Sensor für Stoffwerte | 12 |
| Verbindungsleitung zu 10,11,12 | 13 |
| A/D-Wandler | 14 |
| Prozessor | 15 |
| Regelventile | 16,17,18 |
| Abzweigleitungen | 19,20,21 |
| Regelorgan | 22 |
| Behältnis | 23 |
| Deckel für 23 | 24 |
| Antriebsaggegat | 25 |
| Rührwelle | 26 |
| Rührkörper | 27 |
| Zuleitung | 28,29,30,31,32,33 |
| Innerer Mantel von 23 | 34 |
| Begasungskorb | 35 |
| Öffnungen | 36 |
| Verbindungsleitung | 37 |
| Gaskanal | 38,39,40 |
| Abzweigkanal | 41,42,43 |
| Drehdurchführung | 44 |
| zentraler Gaszuführungskanal | 45 |
| Drehdurchführung | 49 |
| Durchflussmesser für Gas p_{G} | 50 |
| Druckmesser für Gas p_{G} | 51 |
| Oberseite | O |
| Begasungsebene | I, II, III |
| Gasdruck | p_{G} |
| hydrostatischer Druck | pₜ, pₜ₁, pₜ₂, pₜ₃ |
| Tauchtiefe | t₁, t₂, t₃ - |
| Hierzu 3 Blatt Zeichnungen | |

## Patentansprüche

1. Verfahren zum Begasen und Rühren von Stoffen wie Flüssigkeiten, fließfähige Feststoffe, Nährlösungen für Produktbildner, beispielsweise Mikroorganismen, Zellen u. dgl., bei dem der Stoff in einen Reaktorraum eingebracht, mit einer unter Druck stehenden Gasmenge, beispielsweise Luft, Sauerstoff o. dgl., beaufschlagt und zugleich gerührt wird, wobei das Gas in vom Stoffspiegel verschieden hohen vertikalen Stoffsäulen (Begasungsebenen) in den Stoff des Reaktorraums über Begasungskörper zugeführt wird sowie Gas und Stoff durch im Reaktorraum rotierendes Rührkörper durchmischt werden, **gekennzeichnet dadurch, dass** die Menge und der Druck **(p**_{**G**}**)** des zuzuführenden Gases in Abhängigkeit des in den einzelnen Begasungsebenen **(I;II;III)** tatsächlich wirkenden Druckes **(p**_{**t**}**)** und/oder der Stoffeigenschaften des Stoffes dynamisch oder statisch gesteuert wird.

2. Verfahren nach Anspruch 1, **dadurch gekennzeichnet, dass** der Druck **(p**_{**G**}**)** des zuzuführenden Gases auf einen den tatsächlichen Druck **(p**_{**t**}**)** der jeweiligen Stoffsäule überwindenden Wert durch je ein den Begasungsebenen **(I;II;III)** vorgeschaltetes, von einem Prozessor angesteuertes Stellorgan eingestellt wird.

3. Verfahren nach Anspruch 1 und 2, **dadurch gekennzeichnet, dass** der tatsächliche Druck **(p**_{**t**}**)** durch Drucksensoren, die Temperatur und Stoffwerte des Stoffes durch separate Sensoren in den jeweiligen Begasungsebenen **(I;II;III)** ermittelt, in Steuergrößen zur Einstellung der Zustandsgrößen des Gases wie Gasdruck und Gasmenge für das jeweilige Stellorgan und zur Regelung von Prozessparametern wie Rührgeschwindigkeit umgewandelt werden.

4. Verfahren nach Anspruch 1 bis 3, **dadurch gekennzeichnet, dass** der tatsächliche Druck **(p**_{**t**}**)**, die Temperatur und Stoffwerte durch einen den jeweiligen Begasungsebenen **(I;II;III)** zugeordneten gemeinsamen Sensor ermittelt wird.

5. Verfahren nach Anspruch 1 bis 4, **dadurch gekennzeichnet, dass** der Druck **(p**_{**G**}**)** und die Masse des zuführenden Gases vor Zuführung in das Stellorgan mit einem Druckmesser und einem Durchflussmesser ermittelt wird.

6. Verfahren nach Anspruch 1 bis 3, **dadurch gekennzeichnet, dass** der tatsächliche Druck in den jeweiligen Begasungsebenen **(I;II;III)** bei konstantem oder variablen Fullstand gemessen und auf einen den tatsächlichen Druck **(p**_{**t**}**)** der jeweiligen Stoffsäule überwindenden Wert voreingestellt wird.

7. Verfahren nach Anspruch 1 bis 6, **dadurch gekennzeichnet, dass** das Gas außerhalb des Reaktors in eine den Begasungsebenen **(I;II;III)** entsprechende Anzahl von Einzelgasströmen aufgeteilt wird, die den einzelnen Begasungsebenen separat zugeführt werden.

8. Verfahren nach Anspruch 7, **dadurch gekennzeichnet, dass** die Einzelgasströme durch in der Rührwelle vorhandene Kanäle den einzelnen Begasungsebenen **(I;II;III)** zugeführt werden.

9. Verfahren nach Anspruch 7, **dadurch gekennzeichnet, dass** die Einzelgasströme den Begasungsebenen durch ein starr fixiertes Leitungssystem **(I;II;III)** zugeführt werden.

10. Verfahren nach Anspruch 1 und 2, **dadurch gekennzeichnet, dass** das Gas zentral zugeführt und einer sukzessiven Stromteilung mittels einer Druckminderung oder Drosselung in den einzelnen Begasungsebenen **(I;II;III)** und/oder in bzw. an den Begasungskörpern unterworfen wird.

11. Verfahren nach Anspruch 1, 2 und 10, **dadurch gekennzeichnet, dass** die Druckminderung und/oder Drosselung durch Stelleinrichtungen wie Ventile, Blenden, Klappen erzeugt wird.

12. Verfahren nach Anspruch 1, 2 und 10, **dadurch gekennzeichnet, dass** die Druckminderung und/oder Drosselung des Druckes durch eine entsprechend angepasste Gestaltung des Begasungskörpers erzeugt wird.

13. Verfahren nach Anspruch 12, **dadurch gekennzeichnet, dass** zur Anpassung der Begasungskörper poröse Werkstoffe, beispielsweise Sinterwerkstoffe, eingesetzt werden.

14. Vorrichtung zum Begasen und Rühren von Stoffen wie Flüssigkeiten, fließfähige Feststoffe, Nährlösungen für Produktbildner, beispielsweise Mikroorganismen, Zellen u. dgl., mit einem Behältnis als Reaktor zur Aufnahme des Stoffes, einer in das Behältnis geführten Rührwelle mit an dieser befestigten Rührkörpern und hohlen Begasungskörpern, wobei die Begasungskörper in vom Stoffspiegel des Stoffes verschieden beabstandeten Begasungsebenen angeordnet sind, und einer Gaserzeugungseinheit, die Druckgas in die Begasungskörper zur Begasung des Stoffes fördert, **dadurch gekennzeichnet, dass** für die Begasungskörper **(7,7a;8,8a;9,9a)** einer Begasungsebene **(I;II;II)** je eine separate Zuleitung **(28;29;30;31;32;33)** vorgesehen ist, die mit einem separaten Stellorgan **(22)** und einem separaten Durchflussmesser **(50)** zur gesonderten Beaufschlagung einer jeden Leitung **(28;29;30)** mit einem gegenüber den tatsächlichen Druck der Begasungsebene überwindenden Gasdruck **(p**_{**G**}**)** in Verbindung steht.

15. Vorrichtung nach Anspruch 14, **dadurch gekennzeichnet, dass** die Rührwelle **(26)** mit Rührkörpern **(27)** und Begasungskörpern **(7,7a;8,8a;9,9a)** und der Füllstand des Behältnis **(23)** mit Stoff **(6)** in ihrer Lage zueinander im wesentlichen konstant gehalten ist.

16. Vorrichtung nach Anspruch 14, **dadurch gekennzeichnet, dass** die mit den Begasungskörpern **(7,7a;8,8a;9,9a)** verbundenen separaten Zuleitungen durch in der Rührwelle **(26)** angeordnete Gaskanäle **(38;39;40)** und durch an die Gaserzeugungseinheit **(1)** angeschlossene Gaszuleitungen **(28;29;30)** gebildet sind, die mittels einer Drehdurchführung **(44)** an die Gaskanäle angeschlossen sind.

17. Vorrichtung nach Anspruch 14, **dadurch gekennzeichnet, dass** die Drehdurchführung **(44)** innerhalb des Reaktors, aber außerhalb des Stoffes angeordnet ist.

18. Vorrichtung nach Anspruch 14, **dadurch gekennzeichnet, dass** die Drehdurchfuhrung **(44)** von im Reaktor eingefullten Stoff bedeckt ist.

19. Vorrichtung nach Anspruch 14, **dadurch gekennzeichnet, dass** mehrere Drehdurchführungen **(44)** auf der Rührwelle **(26)** angeordnet sind.

20. Vorrichtung nach Anspruch 14 bis 16, **dadurch gekennzeichnet, dass** die mit den Begasungskorpern verbundenen, separaten Zuleitungen **(28;29;30;31;32;33)** innerhalb und außerhalb des Reaktors in einem Begasungskorb **(35)** gehalten sind, wobei die Zuleitungen **(31;32;33)** außerhalb des Behältnis **(23)** zusammengeführt sind und die Zuleitungen **(28;29;30)** mit dem Stellorgan **(16;18;18)** verbunden sind.

21. Vorrichtung nach Anspruch nach Anspruch 16, **dadurch gekennzeichnet, dass** die Zuleitung **(28;29;30)** als eine in der jeweiligen Begasungsebene angeordnete Ringleitung ausgebildet ist.

22. Vorrichtung zum Begasen und Rühren von Stoffen wie Flüssigkeiten, fließfähige Feststoffe, Nährlösungen für Produktbildner, beispielsweise Mikroorganismen, Zellen u. dgl., mit einem Behältnis als Reaktor zur Aufnahme des Stoffes, einer in das Behältnis geführten hohlen Antriebswelle mit an dieser befestigten Rührelementen und hohlen Begasungselementen, wobei die Begasungselemente in vom Stoffspiegel des Stoffes verschieden beabstandeten Begasungsebenen angeordnet sind, und einer Gaserzeugungseinheit, die Druckgas in die Begasungskörper zur Begasung des Stoffes fördert, **dadurch gekennzeichnet, dass** für die Begasungskörper **(7,7a;8,8a;9,9a)** aller Begasungsebenen **(I;II;III)** ein gemeinsamer, durch die Rührwelle **(26)** geführter Gaskanal **(45)** vorgesehen ist, der mit der Gaserzeugungseinheit **(1)** zur Beaufschlagung der einzelnen Begasungskorper **(7,7a;8,8a;9,9a)** mit Gas durch vom Gaskanal **(45)** abzweigende Zufuhrleitungen **(19;20;22)** in Verbindung steht, wobei in bzw. an jeder Zuführleitung **(46;47;48)** ein Stellorgan **(22)** zur Voreinstellung des Gasdruckes auf einen den tatsächlichen Druck der Begasungsebene überwindenden Gasdruck **(p**_{**G**}**)** angeordnet ist.

23. Vorrichtung nach Anspruch 22, **dadurch gekennzeichnet, dass** das Stellorgan **(22)** ein Druckminderer, eine Drosselklappe, eine Blende oder ein poröses Teil ist.

24. Vorrichtung nach Anspruch 14 und 22, **dadurch gekennzeichnet, dass** jeder Begasungsebene **(I;II;III)** ein gemeinsamer Drucksensor **(10)** zur Ermittlung des in der jeweiligen Begasungsebene wirkenden tatsächlichen Druckes **(p**_{**t**}**)** zugeordnet ist.

25. Vorrichtung nach Anspruch 14 und 22, **dadurch gekennzeichnet, dass** jeder Begasungsebene **(I;II;III)** ein Sensor (11) zur Ermittlung der Temperatur des Stoffes zugeordnet ist.

26. Vorrichtung nach Anspruch 14 und 22, **dadurch gekennzeichnet, dass** jeder Begasungsebene **(I;II;III)** ein Sensor **(12)** zur Ermittlung der Stoffwerte der Stoffes zugeordnet ist.

27. Vorrichtung nach Anspruch 14 und 22, **dadurch gekennzeichnet, dass** die Sensoren **(10,;11;12)** in Hohe jeder Begasungsebene **(I;II;III)** an der Innenwand des Behältnis angeordnet sind.

28. Vorrichtung nach Anspruch 14 und 22, **dadurch gekennzeichnet, dass** die Sensoren **(10;11;12)** über einen Analog-Digital-Wandler **(14)**, die Durchflussmesser **(50)** und Druckmesser **(51)** mit einem Prozessor **(15)** zur Ansteuerung der Stellorgane **(16;17;18;22)** verbunden sind.

29. Vorrichtung nach Anspruch 16 bis 23, **dadurch gekennzeichnet, dass** die Begasungskörper **(7,7a;8,8a;9,9a)** hohl und horizontal in der jeweiligen Begasungsebene **(I;II;III)** angeordnet sind und Öffnungen **(36)** für den Austritt des Gases in den Stoff aufweisen.

30. Vorrichtung nach Anspruch 29, **dadurch gekennzeichnet, dass** die Öffnungen **(36)** eine gleiche Form oder unterschiedliche geometrische Formen aufweisen.

31. Vorrichtung nach Anspruch 29, **dadurch gekennzeichnet, dass** die Begasungskörper **(7,7a;8,8a;9,9a)** mit einem auswechselbaren Einsatz versehen sind.

32. Vorrichtung nach Anspruch 14 und 22, **dadurch gekennzeichnet, dass** die Begasungskörper **(7,7a;8,8a;9,9a)** eine hydrodynamisch angepasste Gestalt besitzen.

33. Vorrichtung nach Anspruch 14 und 22, **dadurch gekennzeichnet, dass** Begasungskörper **(7,7a;8,8a;9,9a)** und Rührkörper **(27)** in ihrer Höhe und/oder winklig zueinander versetzt an der Rührwelle **(26)** angeordnet sind.

34. Vorrichtung nach Anspruch 14 und 22, **dadurch gekennzeichnet, dass** die Begasungskörper **(7,7a;8,8a;9,9a)** eine unterschiedliche Länge aufweisen.

35. Vorrichtung nach Anspruch 14 und 22,**dadurch gekennzeichnet, dass** die Rührkörper **(27)** als mehrfach übereinander angeordnete Scheibenrührkörper ausgebildet sind.

36. Vorrichtung nach Anspruch 14 und 22, **dadurch gekennzeichnet, dass** die Ruhrkörper **(27)** gegenuber der Drehrichtung gleichmaßig oder ungleichmäßig winkelig zur Rührwelle **(26)** angestellt sind.

37. Vorrichtung nach Anspruch 14 und 22, **dadurch gekennzeichnet, dass** die Behälterachse des Behältnis **(23)** horizontal oder senkrecht oder in einer Winkellage zur Horizontalachse angeordnet ist.

38. Vorrichtung nach Anspruch 14 und 22, **dadurch gekennzeichnet, dass** die Rührwelle **(26)** horizontal oder senkrecht oder in einer Winkellage zur Horizontalachse angeordnet ist.

39. Vorrichtung nach Anspruch 14 und 22, **dadurch gekennzeichnet, dass** den Rührkörpern **(27)** und Begasungskörpern jeweils getrennte Wellen zugeordnet sind.

40. Vorrichtung nach Anspruch 14 und 22, **dadurch gekennzeichnet, dass** die Rührwelle **(26)** geteilt ausgebildet ist.

41. Vorrichtung nach Anspruch 14 und 22, **dadurch gekennzeichnet, dass** die Rührkörper **(27)** im wesentlichen senkrecht an der Rührwelle **(26)** befestigt und übereinander angeordnet sind.

42. Vorrichtung nach Anspruch 14 und 22, **dadurch gekennzeichnet, dass** jeweils zwischen mindestens zwei Rührkörpern **(27)** ein Begasungskörper **(7,7a;8,8a;9,9a)** angeordnet ist.

43. Vorrichtung nach Anspruch 14 und 22, **dadurch gekennzeichnet, dass** die Rührkörper (27) zugleich als Begasungskörper **(7,7a;8,8a;9,9a)** ausgebildet sind.
